# EUROPEAN PATENT APPLICATION

(11) **EP 3 187 055 A1**
(43) Date of publication of application: **05.07.2017**
(21) Application number: 15827588.3
(22) Date of filing: 13.05.2015
(51) Int. Cl.: A23L 3/32, B65B 55/02, C25B 11/02

(54) **SYSTEM AND METHOD FOR THE STERILIZATION BY ELECTROLYSIS OF THE CONTENT OF CLOSED RECEPTACLES IN CLOSED RECEPTACLES AND CORRESPONDING POST-PACKAGING STERILIZATION RECEPTACLE**

(30) Priority: 01.08.2014 BR 102014019135
(71) Applicant: BCGI Industria de Maquinas e Gestão de Ativos Intangiveis SPE LTDA, 85501-570 Pato Branco PR (BR)
(72) Inventor: DUVOISIN, Charles Adriano, CEP 89280-043 São Bento do Sul, SC (BR)
(74) Representative: Nony
(86) International application number: PCT/BR2015/000066
(87) International publication number: WO 2016/015111

(57) **Abstract**

The present invention is related to a system for sterilization by electrolysis of closed container contents in closed containers and corresponding container for post-packaging sterilization, wherein said sterilization system by electrolysis (100) comprises (i) energizing device (200) having an electrical supply (210), positive pole or anode or positive electrode (220), negative pole or cathode or negative electrode (230), (ii) transport device (300) having movement means (310) or the like and/or transport links (320), and (iii) container (400) having contents (450), at least one anode surface (420), at least one cathode surface (430) and at least one insulating inner surface (440). The present invention is also related to a method for sterilization by electrolysis of container contents closed in closed containers, and to a corresponding container.

## Description

### FIELD OF APPLICATION

The present invention is related to the field of sterilization and/or disinfection of food and utensils by electrolysis.

### INTRODUCTION

The present invention is related to a system and method for sterilization by electrolysis of container contents disposed in closed containers, without significant change of temperature and/or volume of said contents and, thus, decreasing the risk of protein denaturation, wherein said contents are represented by one or more solid or by one or more fluids, isolated or mixed, preferably packed food.

The present invention also relates to a container for post-packaging sterilization.

### STATE OF THE ART

There are different solutions in the state of the art which intend to sterilize both container contents and containers. The sterilization of contents intended for human and/or animal use and/or consumption are especially taken into consideration herein.

A relevant example in the state of the art is the method and system for destruction and/or limiting of microorganism reproduction in nutrient medium, disclosed by the U.S. Patent Document no 3.970.530, which describes the sterilization of an electrically conductive nutrient medium by means of introducing transition metal ions into the medium, through disposal of several galvanic couples on the inner surface of a container containing the nutrient medium. Said galvanic couples have, each, a transition metal anode and a cathode, comprised of at least one metal having a lower standard oxidation potential than that of the transition metal. The solution disclosed by US 3.970 .530 requires disposing metals of different characteristics within the package, wherein galvanic couples benefit oxidation of metals and, consequently, the contamination of the contents. In addition, the disposition of metals for this application type makes the package more expensive, turning it into a low-interest commercial alternative for large-scale usages.

German Patent Application number DE 10 2009 016 821 discloses a device (1) for electric treatment of food (2), comprising at least one container of liquids (3) which can contain a cleaning liquid (4), a direct current supply (5), two electrodes (9a; 9b) and a fluid output (6), by which the cleaning liquid (4) can be guided directly on the food (2) to be treated and/or can be stored in an intermediary manner in a regulation tank. Said Patent Document further discloses that the food (2) to be treated is in direct electrical contact with the cleaning liquid (4). DE 10 2009 016 821 contemplates the direct pouring of the cleaning liquid (4) on the food (2) or its intermediate storage in a regulation tank, for subsequent immersion of the food (2) to be treated, which limits its application for higher volume conditions of the element to be treated and the amount of that same element. In addition, supply is in direct current, which requires the use of specific electric power sources. Finally, said solution is not for sterilization of small containers, let alone for closed containers.

British document GB 2 282 052 discloses the electroheating of food products using low-frequency current, through heating, pasteurization or sterilization of liquid egg products, in an electroheating cell in low voltage and frequency of 60 Hz. Said document describes a solution which, as well as the previous solutions, does not provide nor allow sterilization in small containers, even worse, in closed containers. In addition, the food can experience heating, an effect often unwanted in the food industry, and which is also potentially damaging to the food awaiting sterilization.

Other relevant documents such as, for example, WO 2012/061139, JP3925619, US1,923,421, EP 2 261 169 and US 4.139.348 feature solutions that do not allow sterilization by electrolysis of contents disposed in closed containers, without significant temperature change and/or volume of said contents, i.e. without changing physical and chemical conditions of the contents in question and, in the case of proteins, decreasing the risk of denaturation.

Therefore, such system and method for sterilization by electrolysis of container contents disposed in closed containers, without significant change of temperature and/or volume of said contents and, thus, decreasing the risk of protein denaturation, wherein said contents are represented by one or more solid or by one or more fluids, isolated or mixed, preferably packed food, are relevant.

The same goes for a corresponding container that allows appropriate post-packaging sterilization.

### OBJECTIVE OF THE INVENTION

Therefore, the present invention aims to provide a system for sterilization by electrolysis according to what is claimed in Independent Claim 1 from the set of claims attached.

Another objective of the present invention is to provide a method for sterilization by electrolysis according to Independent Claim 22.

Finally, another objective of this invention is to provide a container for post-packaging sterilization, according to Independent Claim 24.

### BRIEF DESCRIPTION OF THE DRAWINGS

For better understanding and visualization of the object of the present invention, it will now be described with reference to the attached drawings, representing the technical effect obtained by means of exemplary embodiments, which do not limit the scope of the present invention, in which, schematically:

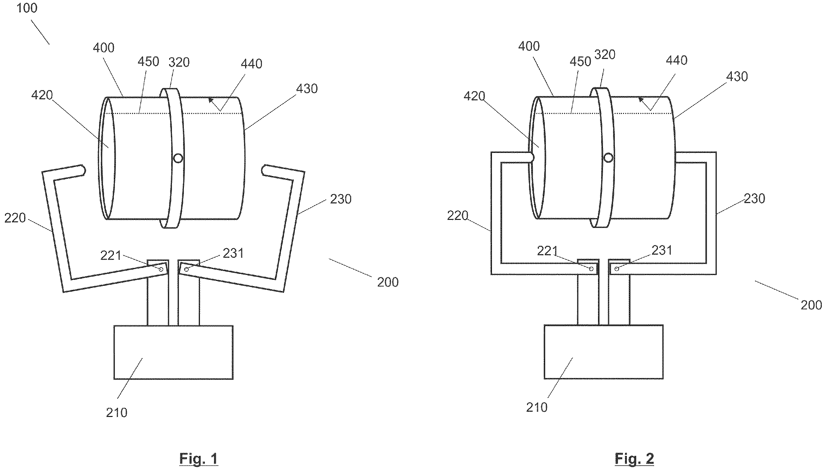

Figure 1: depicts a perspective view of a system for sterilization by electrolysis according to the invention;
Figure 2: is a perspective view of a system for sterilization by electrolysis according to the invention;
Figure 3: depicts a side view of a package according to the invention;
Figure 4: depicts a top view of the package in Figure 3 ;
Figure 5: is a lower view of the package in Figure 3 ;
Figure 6: shows a side view of a package according to the invention;
Figure 7: is a lower view of the package in Figure 6 ;
Figure 8: depicts a side view of a package according to the invention;
Figure 9: is a top view of the package in Figure 8 ;
Figure 10: is a lower view of the package in Figure 8 ;
Figure 11: depicts a side view of a package according to the invention;
Figure 12: is a top view of the package in Figure 11;
Figure 13: shows a lower view of the package in Figure 11;
Figure 14: depicts a perspective view of a system for sterilization by electrolysis according to the invention;
Figure 15: depicts a perspective view of a system for sterilization by electrolysis according to the invention;
Figure 16: depicts a perspective view of a system for sterilization by electrolysis according to the invention;
Figure 17: depicts a perspective view of a system for sterilization by electrolysis according to the invention; and
Figure 18: depicts a perspective view of a packaging line having a system for sterilization by electrolysis according to the invention;

### DETAILED DESCRIPTION OF THE INVENTION

### System for sterilization by electrolysis (100)

As can be inferred from the figures attached, the system for sterilization by electrolysis (100) according to the invention comprises:
(i) at least one energizing device (200) having an electrical supply (210), at least one positive pole or anode or positive electrode (220), at least one negative pole or cathode or negative electrode (230);
(ii) one transport device (300) having movement means (310) or the like and/or transport links (320); and
(iii) at least one container (400) having contents (450), of at least one anode surface (420), at least one cathode surface (430) and at least one insulating inner surface (440).

The energizing device (200) comprises at least one electrical supply (210), at least one positive electrode (220) and at least one negative electrode (230).

The positive electrode (220) and the negative electrode (230) can be, for example, pivotable elements that spin, respectively, around spinning points (221, 231).

In a preferred embodiment of the invention, the energizing device (200) is disposed to allow the conveyance of one or more transport devices (300) that conveys one or more containers (400).

The transport device (300) comprises a movement means (310) that can be, for example, a conveyor or similar and/or be provided with transport links (320) for the transport of one or more containers (400), in order to convey said containers (400) by the vicinity of the energizing device (200).

The container (400), filled with certain contents (450) and sealed, comprises at least one conductive anode surface (420), at least one conductive cathode surface (430), and at least one insulating inner surface (440), non-conductive, and the container (400) may be made of metal, plastic, cardboard, or any usual material known in the package industry for food products, the same goes for its shape or configuration.

In a preferred embodiment of the invention, the contents (450) are a food, represented by one or more solids and/or one or more fluids, isolated or mixed, preferably a packed food.

In a preferred embodiment of the invention, the container (400) is produced entirely of metal, preferably stainless steel, having always an insulating inner surface (440).

The anode (420) and cathode (430) surfaces are opposite to each other, preferably parallel to each other and separated by an insulating inner surface (440), i.e. there is no electrical contact between the anode surface (420) and the cathode surface (430).

In a preferred embodiment of the invention, anode (420) and cathode (430) surfaces have conductive material only in the part facing the inside of the container (400), i.e. they are constructed to form conductive inner faces of the container (400).

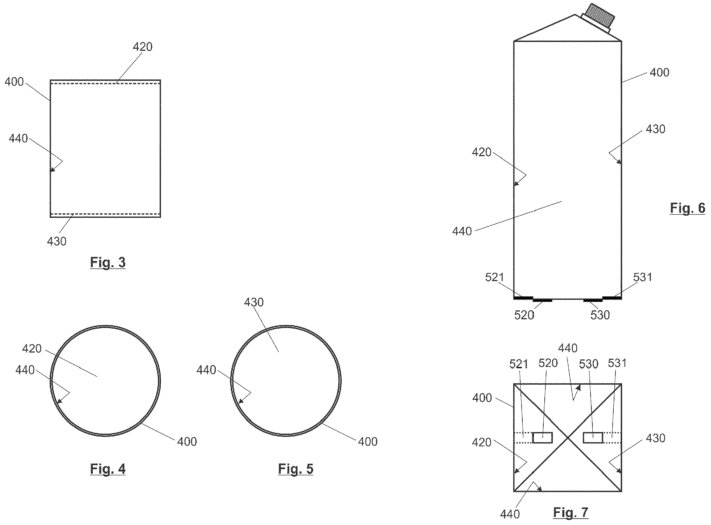

In a container (400) configured in the form of a conventional cylindrical package, for example, a typical and traditional can of canned food, its own upper face takes the role of an anode surface (420) and its own lower face takes the role of a cathode surface (430), wherein these two faces make up the parallel faces of a container (400) of a system (100) according to the invention, whereas the own internal surface of the can takes the role of an insulating inner surface (440) while keeping the anode (420) and cathode (430) surfaces separated from each other, as can be inferred particularly from figures 3, 4 and 5.

The contents (450), in turn take the role of an electrolyte and, in a preferred embodiment of the invention, the electrolyte has an acid activator, for example, but not limited to, acetic acid, grape juice or the like and in concentration depending on the type of contents (450), and, preferably, with taste and odor characteristics which will not alter or negatively affect the taste and odor of the contents (450).

In a preferred embodiment of the invention, the contents (450) have an alkaline activator, preferably with taste and odor characteristics, which will not alter or negatively affect the taste and odor of the contents (450).

In another preferred embodiment of the invention, the contents (450) have a saline activator, in the form of a saline solution, preferably with taste and odor characteristics, which will not alter or negatively affect the taste and odor of the contents (450).

In another preferred embodiment of the invention, the packaging (400) is produced in any suitable and common material in the packaging industry having, however, an internal coating, preferably metallic, especially stainless steel, both at the anode surface (420) and the cathode surface (430), with an element of contact between the above and the external environment to allow the energizing of the above by a positive electrode (220) and a negative electrode (230), respectively.

In a preferred embodiment of the invention, the coating of the anode (420) and cathode (430) surfaces is produced by means of painting with ink or base or a stainless steel-based coating.

In another preferred embodiment of the invention, the coating of the anode (420) and cathode (430) surfaces is produced in galvanized aluminum.

In another preferred embodiment of the invention, the coating of the anode (420) and cathode (430) surfaces is produced in graphite.

In a packaging of this type, represented by a conventional parallelepiped container (400) for example, the traditional packaging for juices, milk and the like, as can be inferred particularly from figures 6 and 7, two alternating faces of the four faces of the container (400) are preferably coated with metal or metalized, representing an anode surface (420) and a cathode surface (430). The faces adjacent to the anode surface (420) and cathode surface (430) are preferably coated with insulating material to prevent direct electrical contact between the anode surface (420) and cathode surface (430). In order to allow electrical contact of the anode (420) and cathode (430) surfaces with their respective positive (220) and negative (230) electrodes, the container (400) has external contact elements (520, 530) having a direct connection by contact with internal contact elements (521, 531) having, in turn, a direct connection, also by contact, with anode (420) and cathode (430) surfaces.

In view of the above conditions, for any type of package, there will be an electrolytic system formed per package (400), anode surface (420), cathode surface (430) and contents (450), wherein the anode surface (420) is an extension of the positive electrode (220), the surface of cathode (430) is an extension of the negative electrode (230) and, as described above, the contents (450) are the electrolyte.

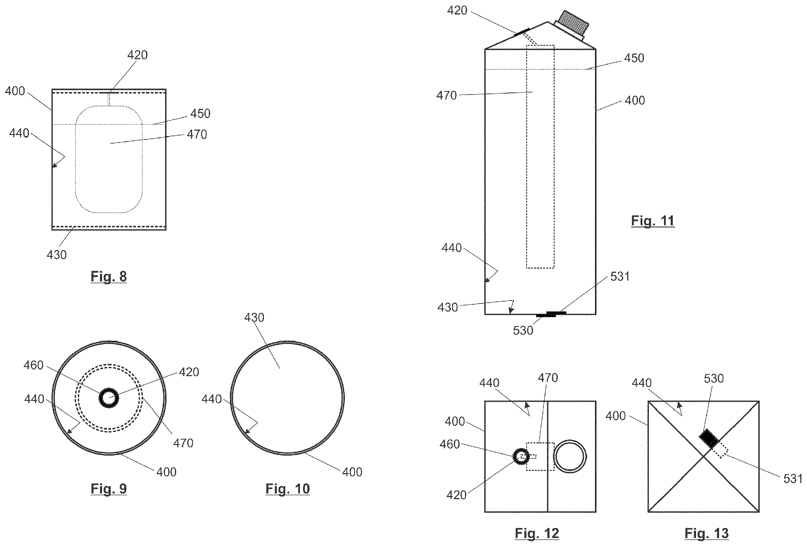

In a preferred embodiment of the invention, as represented by the figures 8, 9 and 10, the anode surface (420) is isolated from its respective face of the packaging (400) by means of an isolating portion (460) and has an immersion rod (470) produced, preferably in a material similar to that of the anode surface (420), that is, in an electrically conductive material, preferably in stainless steel. The immersion rod (470) is immersed in the contents (450) of the packaging (400), but must not touch the cathode surface (430).

In view of the above conditions, there is, again, an electrolytic system formed by package (400), anode surface (420) and immersion rod (470), cathode surface (430) and contents (450).

In another preferred embodiment of the invention, as represented by the figures 11, 12 and 13, the anode surface (420) is isolated from its respective face of the packaging (400) by means of an isolating portion (460) and has an immersion rod (470) produced, preferably in a material similar to that of the anode surface (420), that is, in an electrically conductive material, preferably in stainless steel. The immersion rod (470) is immersed in the contents (450) of the packaging (400), but must not touch the cathode surface (430). In order to allow electrical contact of the cathode surface (430) with the respective negative electrode (230), the container (400) has an external contact element (530) having a direct connection by contact to an element of internal contact (531), which in turn has a direct connection, also by contact, with the respective cathode surface (430).

In view of the above conditions, there is, again, an electrolytic system formed by package (400), anode surface (420) and immersion rod (470), cathode surface (430) and contents (450).

In a preferred embodiment of the invention, one of the electrodes (220, 230) is a penetrating electrode (235), exemplified here, in an illustrative and not limiting way as anode, wherein said penetration anode (235) penetrates the packaging (400) by means of a sealing element (500), being immersed in the contents (450), and thus establishing the electrolysis conditions mentioned above.

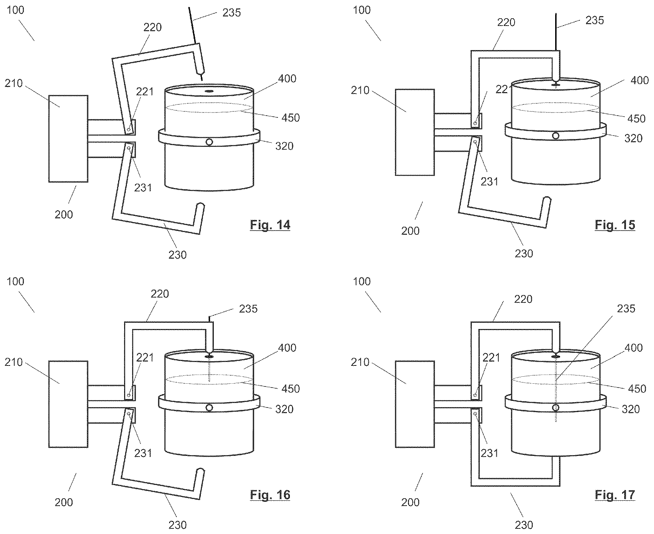

In this variant of anode being in the form of penetration anode (235), said penetration anode (235) must be sharp and thin and the sealing element (500) should be, preferably, a resilient element capable of allowing the conveyance of the penetration anode (235) and the restoration or closing of the opening created by the penetration anode (235) after completion of the sterilization procedure and removal thereof from inside the package (400). A possible sequence of the procedure of inserting a penetration anode (235) through a sealing element (500) is represented by Figures 14, 15, 16, and 17.

### Method for sterilization by electrolysis

A method for sterilization by electrolysis according to the invention, using a system for sterilization by electrolysis (100) according to the invention, comprises the following steps:
Step 1: Packaging of contents (450) in a package (400);
Step 2: Closing of the package (400);
Step 3: Transportation of the package (400) by a transport device (300);
Step 4: Conveyance of the package by an energizing device (200) or station having a power device (200);
Step 5: Establishment of contact of a negative pole or cathode (230) with the cathode surface (430) of the package (400);
Step 6: Establishment of contact of an anode or positive pole (220) with the anode surface (420) of the package (400), optionally of a penetration anode (235) with the contents (450) of the package (400);
Step 7: Application of a direct or alternating electric current, preferably an alternated electric current between 0.1 and 100A, preferably 2A, frequency of 5 Hz to 15 kHz, preferably of 60 Hz with voltage of 12V to 20kV, preferably between 127V and 230V, or frequency of 5 Hz to 15 kHz, preferably of 50 Hz with voltage of 12V to 20kV, preferably 230V, with application times varying between 0.1 second and 300 seconds preferably 45 seconds;
Step 8: Removal of the negative pole or cathode (230) and the positive pole or anode (220), optionally from the penetration anode (235); and
Step 9: Eventual cooling step with time for submission of the package (400) to suitable cooling to the materials involved and to the suitable/recommended temperature range for the contents (450).

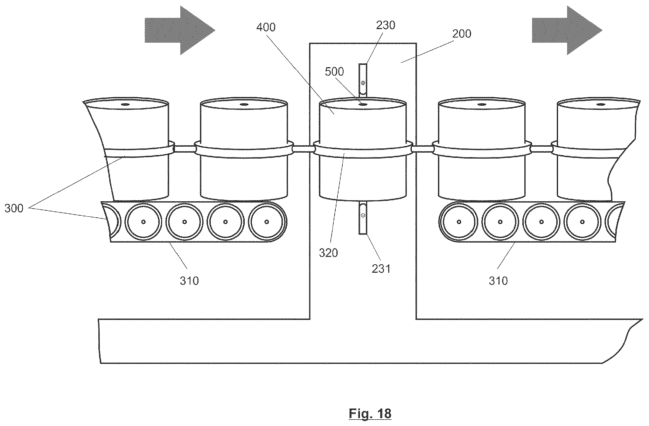

A person skilled in the art will understand easily that the steps 5 and 6 can be reversed according to the type of package or container (400) and/or packaging line available without, however, changing the conditions of electrolysis of the present invention. An example of a packaging line having a system (100) according to the invention is represented schematically by Figure 18.

### Container or package (400) for sterilization by post-packaging electrolysis

A container or a package (400) for sterilization by electrolysis, post-packaging, according to the invention, filled with certain contents (450) and sealed, has at least one conductive anode surface (420), at least one conductive cathode surface (430), and at least one insulating inner surface (440), non-conductive, and the container (400) may be made of metal, plastic, cardboard, or any usual material known in the package industry for food products, the same for its shape or aesthetic configuration.

In a preferred embodiment of the invention, the contents (450) are a food, represented by one or more solids and/or one or more fluids, isolated or mixed, preferably a packed food.

In a preferred embodiment of the invention, the container (400) is produced entirely of metal, preferably stainless steel, having always an insulating inner surface (440).

The anode (420) and cathode (430) surfaces are opposite to each other, preferably parallel to each other and separated by an insulating inner surface (440), i.e. there is no electrical contact between the anode surface (420) and the cathode surface (430).

In a preferred embodiment of the invention, anode (420) and cathode (430) surfaces have conductive material only in the part facing the inside of the container (400), i.e. are constructed to form conductive inner faces of the container (400).

In a preferred embodiment of the invention, the coating of the anode (420) and cathode (430) surfaces is produced by means of painting with ink or base or a stainless steel-based coating.

In another preferred embodiment of the invention, the coating of the anode (420) and cathode (430) surfaces is produced in galvanized aluminum.

In another preferred embodiment of the invention, the coating of the anode (420) and cathode (430) surfaces is produced in graphite.

In a container (400) configured in the form of a conventional cylindrical package, for example, a typical and traditional can of canned foods, its own upper face takes the role of an anode surface (420) and its own lower face takes the role of a cathode surface (430), wherein these two faces make up the parallel faces of a container (400) of a system (100) according to the invention, whereas the can's own internal surface takes the role of an insulating inner surface (440) while keeping the anode (420) and cathode (430) surfaces separated from each other, as can be inferred particularly from figures 3, 4 and 5.

The contents (450), in turn take the role of an electrolyte and, in a preferred embodiment of the invention, the electrolyte has an acid activator, for example, but not limited to, acetic acid, grape juice or the like and in concentration depending on the type of contents (450), and, preferably, with taste and odor characteristics which will not alter or negatively affect the taste and odor of the contents (450).

In a preferred embodiment of the invention, the contents (450) have an alkaline activator, preferably with taste and odor characteristics which will not alter or negatively affect the taste and odor of the contents (450).

In another preferred embodiment of the invention, the contents (450) have a saline activator, in the form of a saline solution, preferably with taste and odor characteristics which will not alter or negatively affect the taste and odor of the contents (450).

In another preferred embodiment of the invention, the package (400) is produced in any suitable and common material in the packaging industry having, however, an internal coating, preferably metallic, especially stainless steel, both at the anode surface (420) and the cathode surface (430), with an element of contact between the above and the external environment to allow the energizing of the above by a positive electrode (220) and a negative electrode (230), respectively.

In a package of such type, represented by a conventional parallelepiped container (400), for example, the traditional packaging for juices, milk and similar, as can be inferred particularly from figures 6 and 7, two alternate faces of the four faces of the container (400) are preferably coated with metal or metallized, representing an anode surface (420) and a cathode surface (430). The faces adjacent to the anode surface (420) and cathode surface (430) are preferably coated with insulating material to prevent direct electrical contact between the anode surface (420) and cathode surface (430).

In order to allow electrical contact of the anode (420) and cathode (430) surfaces with their respective positive (220) and negative (230) electrodes, the container (400) has external contact elements (520, 530) having a direct connection by contact with internal contact elements (521, 531) having, in turn, a direct connection, also by contact, with their respective anode (420) and cathode (430) surfaces.

In a preferred embodiment of the invention, as represented by the figures 8, 9 and 10, the anode surface (420) is isolated from its respective face of the package (400) by means of an isolating portion (460) and has an immersion rod (470) produced, preferably in a material similar to that of the anode surface (420), that is, in an electrically conductive material, preferably in stainless steel. The immersion rod (470) is immersed in the contents (450) of the package (400), but must not touch the cathode surface (430).

In another preferred embodiment of the invention, as represented by the figures 11, 12 and 13, the anode surface (420) is isolated from its respective face of the package (400) by means of an isolating portion (460) and has an immersion rod (470) produced, preferably in a material similar to that of the anode surface (420), that is, in an electrically conductive material, preferably in stainless steel. The immersion rod (470) is immersed in the contents (450) of the package (400), but must not touch the cathode surface (430). In order to allow electrical contact of the cathode surface (430) with the respective negative electrode (230), the container (400) has an external contact element (530) having a direct connection by contact to an element of internal contact (531), which in turn has a direct connection, also by contact, with the respective cathode surface (430).

In the same embodiment represented by figures 11, 12 and 13, it can be concluded that the cathode surface (430) is one of the larger inner side faces of the package (400), instead of being located at the bottom of the package (400).

In a preferred embodiment of the invention, the immersion rod (470) has a dimension such that it is equidistant from the inner walls and the bottom and the top of the container (400).

In a preferred embodiment of the invention, one of the electrodes (220, 230) is a penetrating electrode (235), exemplified here, in an illustrative and not limiting way as anode, wherein said penetration anode (235) penetrates the package (400) by means of a sealing element (500), being immersed in the contents (450).

In this variant of anode being in the form of penetration anode (235), said penetration anode (235) must be sharp and thin and the sealing element (500) should be, preferably, a resilient element capable of allowing the conveyance of the penetration anode (235) and the restoration or closing of the opening created by the penetration anode (235) after completion of the sterilization procedure and removal thereof from inside the package (400).

The present inventive concept can be extended to any type of package, as long as the conditions above are observed. In the case of a canned glass, for example, having metal cover, it is possible to envision the anode surface represented by the metal cover in contact with the positive electrode (220) and the cathode surface represented by the bottom of the glass container having an inner metallic coating and in contact with the negative electrode (230).

### Conclusion

It will be easily understood by those skilled in the art that changes can be made in the present invention without departing from the concepts exposed in the above description. These modifications must be regarded as included in the scope of the present invention. Consequently, the particular embodiments detailed above are only illustrative and exemplary and are non-restrictive as to the scope of the present invention, to which the full extent of the appended set of claims must be observed, and any and all correspondents thereof.

## Claims

1. System for sterilization by electrolysis of closed container contents in closed containers, and corresponding container for post-packaging sterilization, **characterized in that** said system for sterilization by electrolysis (100) comprises:
(i) an energizing device (200) having an electrical supply (210), a positive pole or anode or positive electrode (220), a negative pole or cathode or negative electrode (230);
(ii) a transport device (300) having movement means (310) or the like and/or transport links (320); and
(iii) a container (400) having contents (450), at least one anode surface (420), at least one cathode surface (430) and at least one insulating inner surface (440).

2. System, according to claim 1, **characterized in that** the energizing device (200) is disposed so as to allow the conveyance of one or more transport devices (300) transporting one or more containers (400).

3. System, according to claim 1, **characterized in that** the movement means (310) is a conveyor belt or the like, and **in that** in its movement for conveying, it conveys said containers (400) on the vicinity of the energizing device (200).

4. System, according to claim 1, **characterized in that** the container (400) is produced in metal, plastic, cardboard or a combination thereof.

5. System, according to claims 1 and 4, **characterized in that** the container (400) is produced entirely in metal, preferably stainless steel, invariably having an insulating inner surface (440).

6. System, according to claim 1, **characterized in that** the anode surface (420) and cathode surface (430) are opposite to each other, preferably parallel to each other and separated by an insulating inner surface (440), wherein there is no electrical contact between the anode surface (420) and the cathode surface (430).

7. System, according to claims 1 and 6, **characterized in that** the anode surface (420) and cathode surface (430) are surfaces having conductive material only in the portion facing the inside of the container (400).

8. System, according to claim 1, **characterized in that** the contents (450) are a food represented by one or more solids and/or one or more fluids, isolated or mixed, preferably a packaged food.

9. System, according to claims 1 and 8, **characterized in that** the contents (450) have an acid activator.

10. System, according to claims 1 and 8, **characterized in that** the contents (450) have an alkaline activator,

11. System, according to claims 1 and 8, **characterized in that** the contents (450) have a saline activator, in the form of a saline solution.

12. System, according to claim 1, **characterized in that** the package (400) has an inner coating, preferably metallic, especially stainless steel, both at the anode surface (420) and the cathode surface (430), with a contact element between these and the external environment to allow the energizing thereof by a positive electrode (220) and a negative electrode (230), respectively.

13. System, according to claims 1 and 12, **characterized in that** the coating of the anode surface (420) and cathode surface (430) is produced by means of painting with ink or base or a stainless steel-based coating.

14. System, according to claims 1 and 12, **characterized in that** the coating of the anode surface (420) and cathode surface (430) is produced in galvanized aluminum.

15. System, according to claims 1 and 12, **characterized in that** the coating of the anode surface (420) and cathode surface (430) is produced in graphite.

16. System, according to claim 1, **characterized in that** the anode surface (420) is isolated from the respective face of the package (400) by means of an insulating portion (460), and has an immersion rod (470), preferably produced in similar material to that of the anode surface (420), wherein said immersion rod (470) is to be immersed in the contents (450) of the package (400) but it must not touch the cathode surface (430).

17. System, according to claims 1 and 16, **characterized in that**, in order to allow electrical contact of the cathode surface (430) with the respective negative electrode (230), the container (400) has an external contact element (530) having a direct connection by contact to an element of internal contact (531), which in turn has a direct connection, also by contact, with the respective cathode surface (430).

18. System, according to claim 1, **characterized in that** one of the electrodes (220, 230) is a penetrating electrode (235), wherein said penetration anode (235) penetrates the package (400) through a sealing element (500), being immersed in the contents (450).

19. System, according to claims 1 and 18, **characterized in that** said penetration anode (235) is thin and pointed, and the sealing element (500) is a resilient element capable of allowing the conveyance of the penetration anode (235) and the restoration or closing of the opening created by the penetration anode (235) after completion of the sterilization procedure and the removal thereof from inside the package (400).

20. System, according to claim 1, **characterized in that** the positive electrode (220) and negative electrode (230) are manufactured in metal, preferably stainless steel.

21. System, according to claim 1, **characterized in that** the positive electrode (220) and negative electrode (230) are manufactured in graphite.

22. Method for sterilization by electrolysis of closed container contents in closed containers, and container for post-packaging sterilization thereof **characterized in that** it utilizes a system for sterilization by electrolysis (100) as claimed in claims 1 to 21, and comprising the following steps:
Step 1: Packaging of contents (450) in a package (400);
Step 2: Closing of the package (400);
Step 3: Transportation of the package (400) by a transport device (300);
Step 4: Conveyance of the package by an energizing device (200) or station having a power device (200);
Step 5: Establishment of contact of a negative pole or cathode (230) with the cathode surface (430) of the package (400);
Step 6: Establishment of contact of an anode or positive pole (220) with the anode surface (420) of the package (400), optionally of a penetration anode (235) with the contents (450) of the package (400);
Step 7: Application of a direct or alternating electric current, preferably an alternated electric current between 0.1 and 100A, preferably 2A, frequency of 5 Hz to 15 kHz, preferably of 60 Hz with voltage of 12V to 20kV, preferably between 127V and 230V, or frequency of 5 Hz to 15 kHz, preferably of 50 Hz with voltage of 12V to 20kV, preferably 230V, with application times varying between 0.1 second and 300 seconds, preferably 45 seconds;
Step 8: Removal of the cathode or negative pole (230) and the anode or positive pole (220), optionally from a penetration anode (235); and
Step 9: Eventual cooling step with time for submission of the package (400) to cooling, which is suitable to the materials involved and to the appropriate/recommended temperature range for the contents (450).

23. Method, according to claim 22, **characterized in that** the steps 5 and 6 can be reversed in their sequence of execution, according to the type of package or container (400) and/or package line.

24. Container for post-packaging sterilization, **characterized in that** it is filled with a certain contents (450) and sealed, having at least one anode surface (420), at least one cathode surface (430) and at least one insulating inner surface (440), which is non-conductive.

25. Container, according to claim 24, **characterized in that** the container (400) is produced in metal, plastic, cardboard or a combination thereof.

26. Container, according to claim 24, **characterized in that** the contents (450) are a food represented by one or more solids and/or one or more fluids, isolated or mixed, preferably a packaged food.

27. Container, according to claim 24, **characterized in that** the container (400) is produced entirely in metal, preferably stainless steel, invariably having an insulating inner surface (440).

28. Container, according to claim 24, **characterized in that** the anode surface (420) and cathode surface (430) are opposite to each other, preferably parallel to each other and separated by an insulating inner surface (440), wherein there is no electrical contact between the anode surface (420) and the cathode surface (430).

29. Container, according to claim 24, **characterized in that** the anode surface (420) and cathode surface (430) are surfaces having conductive material only in the portion facing the inside of the container (400).

30. Container, according to claim 24, **characterized in that** the package (400) has an internal coating, preferably metallic, especially stainless steel, both at the anode surface (420) and the cathode surface (430), with a contact element between these and the external environment to allow the energizing thereof by a positive electrode (220) and a negative electrode (230), respectively.

31. Container, according to claims 24 and 30, **characterized in that** the inner coating is produced by means of painting with ink or base or a stainless steel-based coating.

32. Container, according to claims 24 and 30, **characterized in that** the inner coating is produced in galvanized aluminum.

33. Container, according to claim 24, **characterized in that** the anode surface (420) is isolated from the respective face of the package (400) by means of an insulating portion (460) and has an immersion rod (470), preferably produced in similar material to that of the anode surface (420), wherein said immersion rod (470) is to be immersed in the contents (450) of the package (400) but it must not touch the cathode surface (430).

34. Container, according to claims 24 and 33, **characterized in that** the immersion rod (470) has a dimension such that it is equidistant from the inner walls and the bottom and the top of the container (400).

35. Container, according to claim 24 **characterized in that** two alternate faces of the four faces of the container (400) are preferably coated with metal or metallized, representing an anode surface (420) and a cathode surface (430).

36. Container, according to claims 24 and 35, **characterized in that** the faces adjacent to the anode surface (420) and cathode surface (430) are coated with insulating material to prevent direct electrical contact between the anode surface (420) and cathode surface (430).

37. Container, according to claim 24, **characterized in that**, in order to allow electrical contact of the cathode surface (430) with the respective negative electrode (230), the container (400) has an external contact element (530) having a direct connection by contact to an element of internal contact (531), which in turn has a direct connection, also by contact, with the respective cathode surface (430).

38. Container, according to claim 24, **characterized in that** one of the electrodes (220, 230) is a penetrating electrode (235), wherein said penetration anode (235) penetrates the package (400) through a sealing element (500), being immersed in the contents(450).

39. Container, according to claims 24 and 38, **characterized in that** said penetration anode (235) is thin and pointed, and the sealing element (500) is a resilient element capable of allowing the conveyance of the penetration anode (235) and the restoration or closing of the opening created by the penetration anode (235) after completion of the sterilization procedure and the removal thereof from inside the package (400).

40. Container, according to claim 24, **characterized in that** the cathode surface (430) is one of the larger inner side faces of the container (400).

41. Container, according to claim 24, **characterized in that** it is produced in glass.
